# FASCICULE DE BREVET EUROPEEN

(11) **EP 0 722 457 B1**
(45) Date de publication et mention de la délivrance du brevet: **02.01.2002**
(21) Numéro de dépôt: 94929577.8
(22) Date de dépôt: 04.10.1994
(51) Int. Cl.: C07K 5/062, C07K 5/083, C07K 5/103, C07K 7/06, C07K 16/18, A61K 38/05, A61K 38/06, A61K 38/07, A61K 38/08, G01N 33/68

(54) **COMPOSES MODIFIANT LA TRANSMISSION SEROTONINERGIQUE; APPLICATIONS DIAGNOSTIQUES ET THERAPEUTIQUES**
VERBINDUNGEN, DIE DIE SEROTONINERGE ÜBERTRAGUNG BEEINFLUSSEN; IHRE DIAGNOSTISCHEN UND THERAPEUTISCHEN ANWENDUNGEN
COMPOUNDS MODIFYING SEROTONINERGIC TRANSMISSION, DIAGNOSTIC AND THERAPEUTIC APPLICATIONS

(30) Priorité: 04.10.1993 FR 9311804; 09.06.1994 FR 9407078; 09.06.1994 FR 9407079
(43) Date de publication de la demande: 24.07.1996
(73) Titulaire: INSTITUT PASTEUR, 75724 Paris Cédex 15 (FR)
(72) Inventeur: FILLION, Gilles, F-92370 Chaville (FR); ROUSSELLE, Jean-Claude, F-93700 Drancy (FR); MASSOT, Olivier, F-94500 Champigny-sur-Marne (FR)
(74) Mandataire: Le Guen, Gérard
(86) Numéro de dépôt international: FR9401158
(87) Numéro de publication internationale: WO9509868

(56) Documents cités:
- EP-A- 0 017 485
- EP-A- 0 366 638
- WO-A-91/11463
- PHARMACOLOGICAL REVIEWS, vol.44, no.3, Septembre 1992 pages 401 - 458 E. ZIFA AND G. FILLION '5-Hydroxytryptamin Receptors'
- ARCHIVES OF BIOCHEMISTRY AND BIOPHYSICS, vol.276, no.2, 1 Février 1990, SAN DIEGO, US pages 305 - 309 G.D. JOHNSON AND L.B. HERSH 'Studies on the Subsite Specificity of the Rat Brain Puromycin-Sensitive Aminopeptidase'
- DATABASE WPI Section Ch, Week 9210, Derwent Publications Ltd., London, GB; Class B05, AN 92-076744 & JP,A,4 021 635 (SUNTORY LTD)
- DATABASE WPI Section Ch, Week 7534, Derwent Publications Ltd., London, GB; Class B04, AN 75-56264W & JP,A,50 013 373 (DAIGO NUTRITIVE CHEM KK)

## Description

La présente invention a pour objet des composés possédant la propriété de modifier la transmission sérotoninergique ainsi que les applications diagnostiques et thérapeutiques de ces composés.

Le système sérotoninergique cérébral est un système de neurotransmission cérébral et périphérique, qui met en oeuvre la sérotonine ou 5-hydroxytryptamine (5-HT) découverte dans les années 1949-50.

Au niveau central, ce système est particulier, dans la mesure où il est extrêmement centralisé (tous les corps cellulaires sont localisés dans la région postérieure du cerveau, le raphé) et où il se projette dans pratiquement toutes les régions du cerveau. Il est caractérisé par un nombre extrêmement élevé de varicosités (ou équivalent de terminaisons neuronales) le long des axones ; cette disposition multiplie ses points d'interactions avec les autres systèmes neuronaux cérébraux. De plus, une très grande partie de ces varicosités ne présente pas de profil synaptique, c'est-à-dire que la 5-HT libérée par ces "terminaisons" va diffuser et atteindre les cibles (récepteurs) situées à une certaine distance ; ce mécanisme nécessite la mise en oeuvre de récepteurs particuliers possédant une affinité élevée pour l'amine. Cette disposition structurale est tout à fait favorable à une fonction de contrôle de l'ensemble des cellules neuronales du cerveau, et par là à un rôle essentiel dans le maintien de l'homéostasie du système nerveux central.

Pour exercer ce contrôle, le système 5-HT met en oeuvre une grande variété de récepteurs spécifiques. Il s'agit en effet de plusieurs familles dont certaines sont bien caractérisées (5-HT₁, 5-HT₂, 5-HT₃, 5-HT₄) et d'autres mises en évidence plus récemment sont encore peu connues (5-HT₅, 5-HT₆, 5-HT₇). Certaines d'entre elles sont représentées par de nombreux sous-types ; c'est le cas en particulier de la famille 5-HT₁, caractérisée par une affinité nanomolaire de la 5-HT pour ces sites, et qui comporte les sous-types 5-HT_{1A.B.D.E.F.}

Les récepteurs de type 5-HT_{1B} et 5-HT_{1D} sont localisés sur les terminaisons sérotoninergiques et non-sérotoninergiques, où ils modulent la libération du neurotransmetteur contenu dans ces terminaisons, en particulier ils peuvent réguler la libération de la 5-HT elle-même (autorécepteurs) et jouer alors un rôle déterminant dans l'activité de la neurotransmission sérotoninergique.

D'un point de vue fondamental, le système sérotoninergique est impliqué dans de très nombreuses fonctions physiologiques et dans de nombreuses pathologies, et notamment dans le syndrome dépressif.

La dépression est un désordre psychiatrique encore mal connu malgré les très nombreuses études qui lui ont été consacrées. Cependant, il est maintenant assez clair qu'une très grande partie sinon la totalité des dépressions implique le système sérotoninergique et notamment, il est généralement accepté qu'il existe une relation entre un déficit sérotoninergique et le suicide.

En outre, les médicaments antidépresseurs interagissent pour un grand nombre d'entre eux avec le système sérotoninergique et les plus récents, qui sont les plus efficaces et qui possèdent le moins d'effets indésirables, sont le plus souvent de type "sérotoninergique". Ces substances aboutissent toutes, par des mécanismes divers, à faciliter la transmission sérotoninergique (inhibiteurs de monoamine oxydases, inhibiteurs de recapture, agonistes 5-HT_{1A}). Mais d'une part, leur efficacité est limitée, et d'autre part, ils possèdent encore pour nombre d'entre eux des effets secondaires importants, et enfin, leur délai d'action est important (> 2 semaines).

Les résultats récents de la littérature et des inventeurs indiquent que les récepteurs 5-HT_{1B/1D} pourraient jouer un rôle important dans le syndrome dépressif et dans le mécanisme d'action des antidépresseurs. Il a en effet été montré que non seulement ils modulent la libération d'acétylcholine, mais ils modulent aussi celle de la 5-HT elle-même, et par là même régulent de façon efficace la transmission sérotoninergique dont on sait l'importance dans le syndrome dépressif. De plus, ils sont la cible des antidépresseurs qui interagissent de façon vraisemblablement non compétitive ; autrement dit, les antidépresseurs reconnaîtraient un site distinct des récepteurs 5-HT_{1B/1D}, mais étroitement interactif avec ces derniers.

Les travaux des inventeurs ont permis la mise en évidence d'un composé endogène cérébral reconnaissant un site récepteur capable d'interagir avec le fonctionnement de certains récepteurs 5-HT, en particulier les récepteurs 5-HT_{1B/1D} qui modulent la libération de la 5-HT elle-même.

Le composé endogène cérébral mis en évidence est un tétrapeptide de séquence :

Leu - Ser - Ala - Leu (séquence LSAL).

Les inventeurs ont également testé l'activité de peptides comprenant une partie de la séquence LSAL ou la séquence LSAL modifiée et montré que ces composés avaient encore une bonne activité sur les récepteurs 5-HT_{1B/1D}. De manière générale, une modification (par exemple une acétylation) de l'extrémité NH₂ terminale aboutit à un composé ayant une moins bonne activité de ligand sur le récepteur 5-HT, alors que des modifications de l'extrémité COOH permettent d'obtenir un peptide qui présente encore une activité importante.

Par exemple, l'adjonction de la séquence Gly-Gly-Gly-Tyr à l'extrémité COOH de la séquence LSAL permet d'obtenir un composé ayant une activité encore importante. Cependant, l'adjonction de la même séquence, mais dont le résidu Tyr est iodé aboutit à un composé n'ayant plus d'activité de ligand du récepteur 5-HT, probablement en raison de l'encombrement stérique dû à la taille importante des atomes d'iode qui empêche la liaison du peptide ainsi modifié sur son récepteur.

L'invention a ainsi pour objet un composé de séquence peptidique X Leu Y dans laquelle X représente H ou Ala ou Leu-Ser-Ala, Y représente une séquence peptidique ayant de 1 à 10 aminoacides, dont l'extrémité carboxy terminale est amidifiée par un groupe NH₂ ou estérifiée par un reste hydroxycarbyloxy substitué ou non substitué,
ou -Ala-Leu-Ser, ou -Leu-Ser-Ala-Leu-Z, dans laquelle Z représente OH, NH₂, un reste hydroxycarboxy substitué ou non substitué, ou une séquence peptidique ayant de 1 à 10 aminoacides,
ainsi que les composés correspondants dans lesquels la liaison peptidique -CO-NH- est remplacée par une liaison résistante à la dégradation enzymatique des protéases, ou dans lesquels le squelette peptidique comporte un ou plusieurs groupes intercalés rendant la liaison peptidique résistante à la dégradation enzymatique.

Ainsi, le groupe de liaison peptidique (-CO-NH-) est avantageusement remplacé par un groupe de liaison -CO-NR'-, -CR₁R₂-CR₃R₄-, -CO-CR₁R₂-, R₁, R₂, R₃ et R₄, identiques ou différents, représentant H ou un groupe alkyle en C₁-C₆, notamment un groupe méthyle, et R' représentant un groupe alkyle en C₁-C₆.

Parmi les groupes pouvant être intercalés dans le squelette peptidique, on peut citer les groupes suivants -CR₁R₂-, -NR₁- et -O-, R₁ et R₂ étant définis comme précédemment.

Par reste hydrocarbyloxy, on entend notamment un groupe alkyloxy, alcènyloxy ou alcynyloxy, à chaîne linéaire ou ramifiée, ayant de 1 à 10 atomes de carbone non substitué ou substitué par un ou plusieurs groupes choisis parmi OH, NH₂, NO₂, Cl, Br, F, CF₃, par exemple le groupe OCH₃, OC₂H₅, OCHOH-CH₃, OCHOH-CH₂OH, OCH₂CH₂NH₂, OC₃H₇, etc...

L'invention a également pour objet l'utilisation d'un composé de séquence peptidique suivante : - X Leu Y dans laquelle X représente H ou Ala ou Leu-Ser-Ala, Y représente OH, ou une séquence peptidique ayant de 1 à 10 aminoacides, dont l'extrémité carboxy terminale est amidifiée par un groupe NH₂ ou estérifiée par un reste hydroxycarbyloxy substitué ou non substitué,
sous réserve que lorsque X représente H, Y ne représente pas OH,
ou -Ala-Leu-Ser, ou -Leu-Ser-Ala-Leu-Z dans laquelle Z représente OH, NH₂, un reste hydroxycarbyloxy substitué ou non substitué, ou une séquence peptidique ayant de 1 à 10 aminoacides,
- ainsi que les composés correspondants dans lesquels la liaison -CO-NH- peptidique est remplacée par une liaison résistante à la dégradation enzymatique des protéases ou dans lesquels la squelette peptidique comporte un ou plusieurs groupes intercalés rendant la liaison peptidique résistante à la dégradation enzymatique, pour la préparation d'une composition ayant une activité modulatrice de la transmission sérotoninergique.

Les composés selon l'invention comprennent notamment les peptides de séquence suivante : Leu - Ser - Ala - Leu ; Leu - Ser - Ala - Leu - OCH3 ; Leu - Ser - Ala - Leu - NH2 ; 
Leu - Ser - Ala - Leu - Gly - Gly - Gly -
Tyr ;
 ainsi que les composés analogues dans lesquels la liaison peptidique est remplacée par une liaison telle que définie ci-dessus, ou dont le squelette comprend un groupe intercalé tel que défini ci-dessus.

Les composés les plus actifs sont ceux dont les aminoacides constitutifs sont sous la forme L, c'est à dire la forme naturelle pour les structures peptidiques définies ci-dessus.

Les composés selon l'invention sont préparés par extraction à partir de cerveau lyophilisé comme décrit ci-après ou plus avantageusement par synthèse peptidique classique, par exemple par la méthode en phase solide de MERRIFIELD lorsqu'ils sont de nature exclusivement peptidique, ou encore par modification des groupes terminaux des aminoacides et condensation des résidus ainsi obtenus, les fonctions réactives autres que celles engagées dans la liaison entre les résidus successifs ayant été préalablement protégées à l'aide de groupes protecteurs bien connus de l'homme du métier.

Selon un exemple de préparation d'un peptide selon l'invention, on fixe sur une résine, par l'intermédiaire de son groupe carboxylique, le premier résidu de l'extrémité COOH terminale dont la fonction amine est protégée par un groupe terbutyloxycarbonyle, puis, après avoir déprotégé la fonction amine en lavant la résine avec de l'acide trifluoroacétique dans du dichlorométhane, on couple dans le diméthylformamide le second résidu d'acide aminé dont la fonction amino est protégée comme précédemment, on fixe ainsi les uns après les autres les résidus aminoacides qui vont constituer la portion du peptide selon l'invention. Après déprotection, la fonction amine du résidu N-terminal peut être acétylée par action d'un excès d'anhydride acétique en présence de diisopropyléthylamine.

Les chaînes latérales réactives des aminoacides peuvent être protégées par exemple par un groupe benzyle, pour les chaînes latérales hydroxyles.

Après avoir éliminé le groupe protecteur, le peptide selon l'invention est libéré du support solide, par exemple avec de l'acide fluorhydrique. Le produit brut est lyophilisé et soumis à une chromatographie en phase liquide à moyenne pression, permettant d'obtenir un produit pratiquement pur ; celui-ci est alors caractérisé par chromatographie en phase liquide à haute pression ainsi que par l'analyse de sa composition en aminoacides et par spectrométrie de masse.

Les peptides caractérisés possèdent la propriété de modifier la transmission sérotoninergique par ses interactions avec les autorécepteurs (et aussi les hétérorécepteurs) et jouent un rôle clef dans diverses pathologies psychiatriques où le système 5-HT est notoirement mis en jeu (stress, anxiété, dépression, obsessions compulsives, troubles de l'appétit, du sommeil, troubles du comportement, agressivité, etc...).

L'invention a également pour objet une composition thérapeutique comprenant un composé tel que défini ci-dessus, capable de traverser la barrière hématoencéphalique.

Pour traverser la barrière hématoencéphalique, le peptide est de préférence administré sous la forme d'une pro-drogue, notamment de type glycosylphospho-triester, dans laquelle le peptide est lié par exemple par la leucine au groupe phosphate, comme décrit dans J. MED. CHEM. 92, Vol. 35, p. 30-39.

Une telle pro-drogue, ainsi que de manière générale une molécule précurseur susceptible de libérer in vivo un composé selon l'invention, consitue un autre objet de l'invention.

La composition thérapeutique est utilisée avantageusement dans des pathologies dans lesquelles est impliqué le système sérotoninergique, notamment dans les pathologies liées à une déficience de la transmission sérotoninergique.

Ainsi, la composition selon l'invention peut être utilisée en particulier dans le traitement de la dépression, mais également dans toutes les indications actuellement couvertes par les antidépresseurs, comme les troubles obsessionnels compulsifs, l'anxiété généralisée, la crise de panique, les troubles de l'appétit, du sommeil, l'impulsivité, les troubles sexuels, l'agressivité, et plus généralement celles des composés facilitant la transmission sérotoninergique.

La composition selon l'invention se présente avantageusement sous forme injectable ou orale, ou sous toute autre forme pharmaceutique connue.

La dose thérapeutique efficace est aisément déterminée par l'homme du métier en fonction de la nature et de la gravité de la pathologie à traiter, ainsi que du poids et de l'âge du patient. Les composés selon l'invention ne sont pas toxiques. Ils sont ajoutés à un véhicule pharmaceutiquement acceptable ou à un excipient bien connu de l'homme du métier.

L'invention a également pour objet un procédé de diagnostic in vitro d'une affection liée au système sérotoninergique chez un patient, caractérisé en ce que l'on effectue un dosage dans un fluide biologique du patient d'un peptide de séquence

Leu - Ser - Ala - Leu

Le dosage est effectué notamment par une méthode immunologique comprenant la mise en présence d'un prélèvement biologique avec un anticorps spécifiquement dirigé contre un peptide selon l'invention et la mise en évidence du complexe antigène-anticorps ainsi formé.

Ces procédés peuvent être basés sur une méthode radioimmunologique de type RIA, RIPA ou IRMA, ou sur une méthode immunoenzymatique, par exemple de type ELISA.

Le fluide biologique peut être le sang, l'urine ou le liquide céphalo-rachidien.

Les anticorps peuvent être monoclonaux ou polyclonaux et constituent, ainsi que leurs fragments Fab, Fab', F(ab')₂ et Fc, un autre objet de l'invention.

Les anticorps sont obtenus par couplage d'un peptide selon l'invention sur un peptide ou une protéine porteurs immunogènes.

L'invention a également pour objet un kit de diagnostic pour le dosage d'un peptide selon l'invention dans un fluide corporel d'un patient chez lequel on suspecte une pathologie liée à une déficience de la transmission sérotoninergique, notamment une dépression masquée, comprenant au moins un anticorps tel que défini ci-dessus et éventuellement les réactifs pour la mise en oeuvre du dosage.

Les peptides de l'invention se sont révélés inactifs sur le site de recapture de la 5-HT.

L'existence d'un facteur endogène cérébral non actif sur le site de recapture constitue un standard de référence en terme d'activité et permet ainsi d'envisager un procédé de sélection de substances antidépressives, consistant à discriminer entre l'effet de recapture et l'effet d'interaction sur le site 5-HT_{1B/1D}.

Ce procédé s'applique aux antidépresseurs connus et permet également la mise au point de médicaments nouveaux.

En pratique, la discrimination entre l'effet de recapture et l'effet sur le site 5-HT_{1B/1D} peut être effectuée en réalisant des essais d'inhibition de liaison entre d'une part des quantités croissantes d'antidépresseurs et la capture de la 5-HT dans les synaptosomes, la 5-HT étant radiomarquée et d'autre part en réalisant des essais de même type sur le récepteur 5-HT_{1B/1D} en faisant varier la quantité d'antidépresseurs en présence d'un ligand radiomarqué spécifique de ces récepteurs, et en déterminant les Ki respectifs ou de façon plus générale les CI₅₀.

Le choix d'antidépresseurs ayant une activité sélective sur le site 5-HT_{1B/1D} sera dicté par les substances présentant un Ki élevé ou une CI₅₀ faible dans l'essai de recapture et inversement un Ki faible ou une CI₅₀ élevée dans l'essai d'inhibition de liaison sur le site de reconnaissance du peptide du récepteur 5-HT_{1B/1D}. On peut également et plus aisément utiliser le peptide radiomarqué (tritium) pour identifier et caractériser le site spécifique de reconnaissance du peptide, et ainsi directement étudier les capacités de diverses substances à déplacer (compétition) ce peptide en mesurant leur Ki et en comparant ce dernier aux valeurs de Ki obtenues pour ces mêmes substances pour inhiber la capture de la 5-HT (ou déplacer un inhibiteur de recapture (³H paroxetine par exemple).

Un autre objet de l'invention consiste également en un procédé de mise en évidence de ligands du site du récepteur 5-HT_{1B/1D} pour le peptide LSAL, caractérisé en ce que l'on effectue les étapes consistant à :
- mettre en contact une molécule ou un mélange contenant différentes molécules, éventuellement non identifiées avec une cellule recombinée exprimant à sa surface le récepteur 5-HT_{1B/1D} en présence d'un composé selon l'invention dans des conditions permettant l'interaction entre le récepteur 5-HT_{1B/1D} et ladite (lesdites) molécule(s), dans le cas où celle-ci possèderait une affinité pour le récepteur 5-HT_{1B/1D};
- détecter la quantité dudit composé selon l'invention liée au récepteur;
- en déduire la fixation éventuelle de ladite (desdites) molécule(s).

Ce procédé peut être utilisé pour la mise en évidence d'agonistes ou d'antagonistes du peptide LSAL.

Un autre objet de l'invention concerne un procédé de mise en évidence de modulateurs(antagonistes ou agonistes) du site du récepteur 5-HT_{1B/1D} pour le peptide LSAL caractérisé en ce que l'on effectue les étapes consistant à :
- mettre en contact une molécule ou un mélange contenant différentes molécules, éventuellement non identifiées, avec une cellule recombinée exprimant à sa surface le récepteur 5-HT_{1B/1D} en présence d'un composé selon l'invention, dans des conditions permettant l'interaction entre le récepteur 5-HT_{1B/1D} et ledit composé selon l'invention;
- détecter les molécules capables de mimer ou d'antagoniser l'activité du composé selon l'invention sur ledit récepteur.

Des ligands obtenus selon un procédé tel que décrit ci-dessus peuvent être utilisés comme principe actif d'une composition thérapeutique utile dans le traitement de troubles psychiatriques tels que la dépression, circulatoires tels que la migraine, ou immunologiques liés aux récepteurs 5-HT_{1B/1D}.

Les composés selon l'invention sont également utiles comme outils de diagnostic pour marquer les récepteurs 5-HT_{1B/1D}, et plus particulièrement le site de liaison du peptide endogène sur ces récepteurs.

A cette fin, on utilise avantageusement une composition de diagnostic comprenant un composé selon l'invention radiomarqué dans une technique d'imagerie mettant en oeuvre un scanner de tomographie par émission de positons.

On décrira ci-après l'isolement et la caractérisation du peptide Leu-Ser-Ala-Leu selon l'invention, ainsi que les propriétés de liaison et les activités pharmacologiques sur les récepteurs 5-HT_{1B/1D} du peptide Leu-Ser-Ala-Leu en se reportant aux figures annexées sur lesquelles :
- La Figure 1 représente les interactions de liaison avec le [¹²⁵I]cyanopindolol.
   La liaison du [¹²⁵I]cyanopindolol a été mesurée comme décrit dans HOYER D. et al., Eur. J. Pharmacol. 118, 1-12 (1985).
- La Figure **1A** représente les résultats obtenus avec des membranes corticales de cerveau de rat (50 µg de protéines), incubées pendant 60 minutes à 37°C dans un volume total de 100 µl en présence de [¹²⁵I]cyanopindolol 0,3 nM.
   L'incubation a été effectuée dans un tampon tris-HCl 5 mM, pH 7,5, contenant 154 mM NaCl, 1 µM de pargyline, 30 µM d'isoprotérénol et différentes concentrations du peptide (10⁻¹³ à 10⁻⁸ M). Chaque point correspond à une moyenne plus ou moins l'écart type de cinq déterminations indépendantes.
- La Figure **1B** représente les résultats obtenus avec des membranes de cellules NIH 3T3, transfectées avec le gène du récepteur 5-HT_{1B} (50 µg de protéines), incubées pendant 60 minutes à 37°C en présence de [¹²⁵I]cyanopindolol 0,3 nM. La liaison a été mesurée comme pour la Figure **1A**.
- La Figure **1C** représente les résultats obtenus des membranes corticales de cerveau de rat (50 - µg de protéines) incubées pendant 60 minutes à 37°C dans un volume total de 100 µl en présence de concentrations croissantes de [¹²⁵I]cyanopindolol (0,06 à 0,35 nM) avec (○) ou sans (●), le peptide à 1 nM. Chaque point correspond à la moyenne plus ou moins l'écart type de trois déterminations indépendantes.
- La Figure **1D** représente la courbe de Scatchard des courbes de saturation.
- La Figure **2** représente les résultats des effets de LSAL sur l'activité adénylylcyclase stimulée par la forskoline.

Des membranes de cellules NIH 3T3 exprimant le gène du récepteur 5-HT_{1B} de souris ont été mises en suspension et homogénéisées avec un homogénisateur Dounce dans un tampon tris-HCl 50 mM pH 7,4 contenant MgCl₂ à 3 mM et EGTA à 0,2 mM (tampon TME).

Les activités adénylyl cyclase ont été mesurées dans des fractions aliquotes de 50 µl (30-50 µg de protéines) d'homogénéisats dans un volume final de 100 µl contenant 0,1 mM, [α³²P]ATP (1µCi), 1 mM MgCl₂, 50 µM GTP, 10 µM forskoline, 100 mM NaCl, 10 mM théophylline, 20 mM phosphocréatinine, 0,2 mg/ml créatine kinase, 1 mM [³H]AMPc (≈ 15 000 cpm/essai) et les substances à étudier.

Les membranes ont été incubées pendant 20 minutes à 30°C. La réaction a été arrêtée par addition de 200 µl d'ATP à 5 mM, 5 mM d'AMPc et du dodécyl sulfate de sodium à 1% dissous dans 50 mM de tampon Tris-HCl.

Le [³²P]AMPc formé a été isolé par la méthode de chromatographie à deux colonnes décrite par Salomon et al. (Adv. Cyclic Nucleotide Res., 10, 35-55 (1979)., modifié par De Vivo et Maayani (J. Pharmacol. Exp. Therap. 238, 248-253 (1986)).

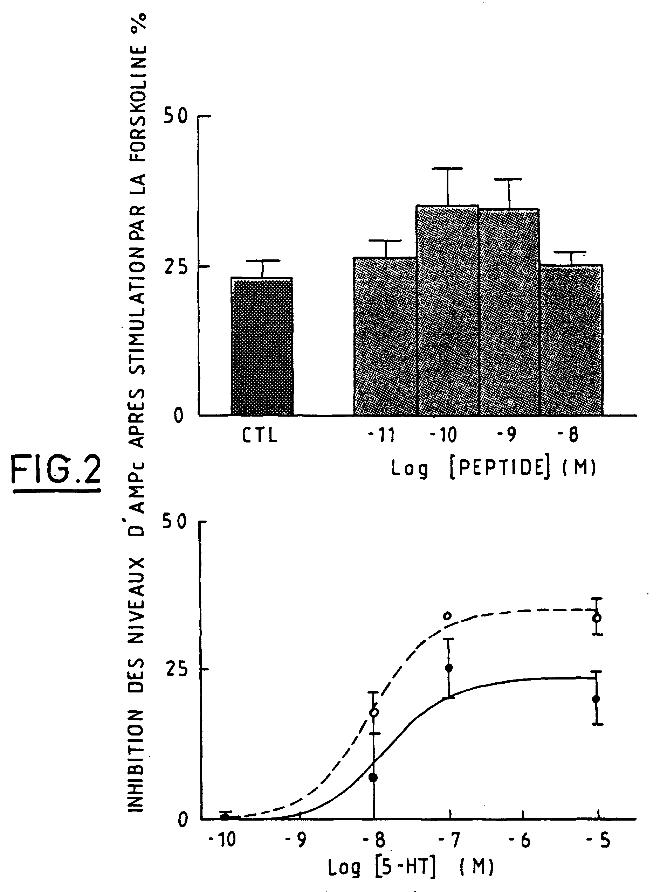

- La Figure **2A** exprime l'effet de LSAL en fonction de sa concentration sur l'activité enzymatique stimulée par la forskoline en présence de 0,1 µM de 5-HT.
- La Figure **2B** exprime la courbe dose-réponse de l'effet de 5-HT sur l'activité enzymatique stimulée par la forskoline en l'absence (• ---- •), ou en présence (○ ---- ○) de LSAL (10⁻⁹ M).

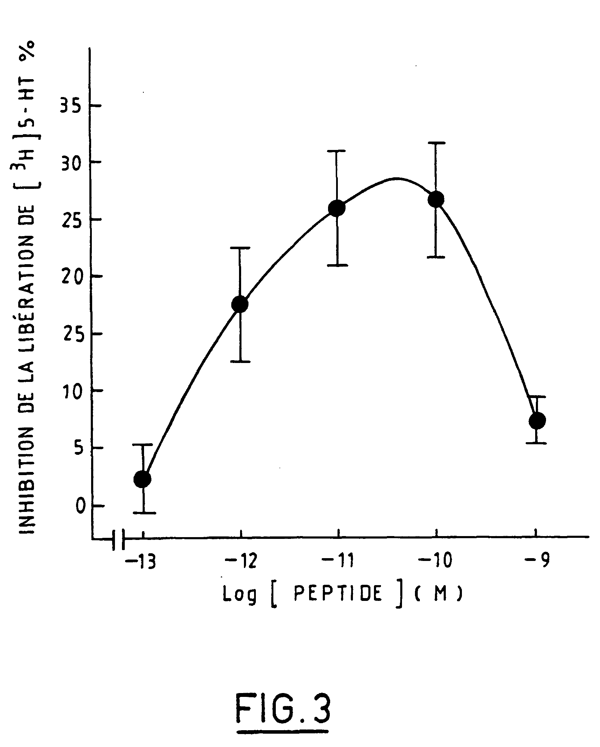

- La Figure **3** représente l'effet du peptide sur la libération de sérotonine dans des synaptosomes de l'hippocampe de cerveau de rat. Les résultats sont exprimés en pourcentages de la libération de [³H]5-HT (rapport P₂/P₁)en l'absence de peptide. Chaque point est la moyenne plus ou moins l'écart type de déterminations en triple obtenue à l'aide de 3-6 homogénéisats d'hippocampe distincts.

La méthode mise en oeuvre est la méthode décrite précédemment (BOLANOS-JIMENEZ B. et al., Eur. J. Pharmacol. 242, 1-6 (1993)) avec les modifications suivantes : la [³H]acétylcholine était substituée par 60 nM [³H]5-HT et 0,01% d'acide ascorbique était ajouté au milieu d'incubation.

### 1. Isolement et purification du peptide Leu-Ser-Ala-Leu

En utilisant comme critère de discrimination la capacité d'un facteur potentiel à interagir avec la reconnaissance de la 5-HT par le récepteur 5-HT_{1B/1D} de membranes de synaptosomes de cerveau de rat, comme décrit dans FR-2 657 784, un composé actif a été purifié à partir d'extraits de cerveaux de rat, de cheval et de bovin. La méthodologie de purification a fait appel à des séparations par perméation sur gel, des phases normales ou inversées ou encore des échanges ioniques.

Elle est résumée sur le tableau ci-dessous :

| **Etapes de purification du peptide endogène** | | |
|---|---|---|
| **ETAPES DE LA CHROMATOGRAPHIE** | **CONDITIONS DE LA CHROMATOGRAPHIE** | **FRACTION ACTIVE (Temps d'élution ou de rétention)** |
| EXCLUSION DE TAILLE (TSK HW 40S, Merck, 600 × 26 mm) | Elution isocratique avec un tampon | T_{E} = 80 min. |
| | d'acétate d'ammonium | |
| | (CH₃COONH₄) 50mM (pH 5) | |
| | Vitesse d'écoulement : 2 ml.min⁻¹ | |
| PHASE INVERSE (C₁₈, Ultrabase, Shandon, 250 × 10 mm) | Gradient d'élution linéaire de A à B | T_{R} = 20 min. |
| | pendant 12 minutes : | |
| | A : 50 mM CH₃ COONH₄ (pH 5) ; | |
| | B : 50 mM CH₃ COONH₄ (pH 5)/CH₃CN, 88/12 | |
| | Gradient par étapes pendant 5 min. avec C. | |
| | C : 50 mM CH₃ COONH₄ (pH 5)/CH₃CN, 50/50 | |
| | Vitesse d'écoulement : 4 ml.min⁻¹ | |
| EXCLUSION DE TAILLE (Sephadex G₂₅, Pharmacia, 450 × 16 mm) | Elution isocratique avec | T_{E} = 240 min. |
| | 10 mM CH₃ COONH₄ (pH 5) | |
| | Vitesse d'écoulement : 0.3 ml.min⁻¹ | |
| | | |
| PHASE INVERSE (C₁₈, Ultrabase, Shandon, 250 × 10 mm) | Gradient d'élution linéaire de A à B | T_{R} = 5.75 min. |
| | pendant 15 minutes | |
| | A : 50 mM CH₃ COONH₄ (pH 5)/CH₃CN, 85/15 | |
| | B : 50 mM CH₃ COONH₄ (pH 5)/CH₃CN, 75/25 | |
| | Gradient par étapes pendant 5 min. avec C. | |
| | C : 50 mM CH₃ COONH₄ (pH 5)/CH₃CN, 50/50 | |
| | Vitesse d'écoulement : 4 ml.min⁻¹ | |
| PHASE INVERSE (C₁₈, Ultrabase, Shandon, 250 x 10 mm) | Gradient d'élution linéaire de A à B | T_{R} = 28 min. |
| | pendant 35 minutes | |
| | A : 50 mM CH₃ COONH₄ (pH 5) : | |
| | B : 50 mM CH₃ COONH₄ (pH 5)/CH₃CN, 70/30 | |
| | Vitesse d'écoulement : 4 ml.min⁻¹ | |
| PHASE PSEUDO-INVERSE (Colonne de charbon, Hypercarb., Shandon, 100 × 3 mm) | Gradient d'élution linéaire de A à B | T_{R} = 12.71 min. |
| | pendant 30 minutes | |
| | A : 50 mM CH₃ COONH₄ (pH 5) : | |
| | B : 50 mM CH₃ COONH₄ (pH 5)/CH₃CN, 70/30 | |
| | Vitesse d'écoulement : 1 ml.min⁻¹ | |
| PHASE INVERSE (C₁₈, Ultrabase, Shandon, 150 × 4 mm) | Elution isocratique avec 0,05% | T_{R} = 5.14 min. |
| | TFA/CH₃CN, 83/17 | |
| | Vitesse d'écoulement : 1 ml.min⁻¹ | |

La caractérisation des fractions obtenues a été réalisée essentiellement sur la base d'analyse par RMN, d'analyse d'aminoacides et de séquençage d'aminoacides. Le composé purifié consistait en Leu-Ser-Ala-Leu.

La même fraction a été obtenue également à partir de cerveau de cheval et de bovin, ce qui signifie que ce peptide est conservé chez les mammifères.

### 2. Interactions du peptide purifié avec les récepteurs 5-HT_{1B/1D}

Le peptide synthétique LSAL a été testé pour sa capacité à "mimer" les effets de la fraction purifiée obtenue à partir d'extraits de cerveau. LSAL inhibe la liaison de [³H]-5-HT aux récepteurs 5-HT₁ avec une CI₅₀ proche de 10⁻¹¹M et un effet inhibiteur maximum de 30 à 50% se produisant à une concentration de 10⁻⁹M. La liaison aux 5-HT_{1nonA}, qui dans les conditions expérimentales mises en oeuvre, représente principalement des sites 5-HT_{1B/1D}, est inhibée jusqu'à une valeur maximale de 75%, comme représenté au tableau 2 ci-dessous avec une CI₅₀ identique.

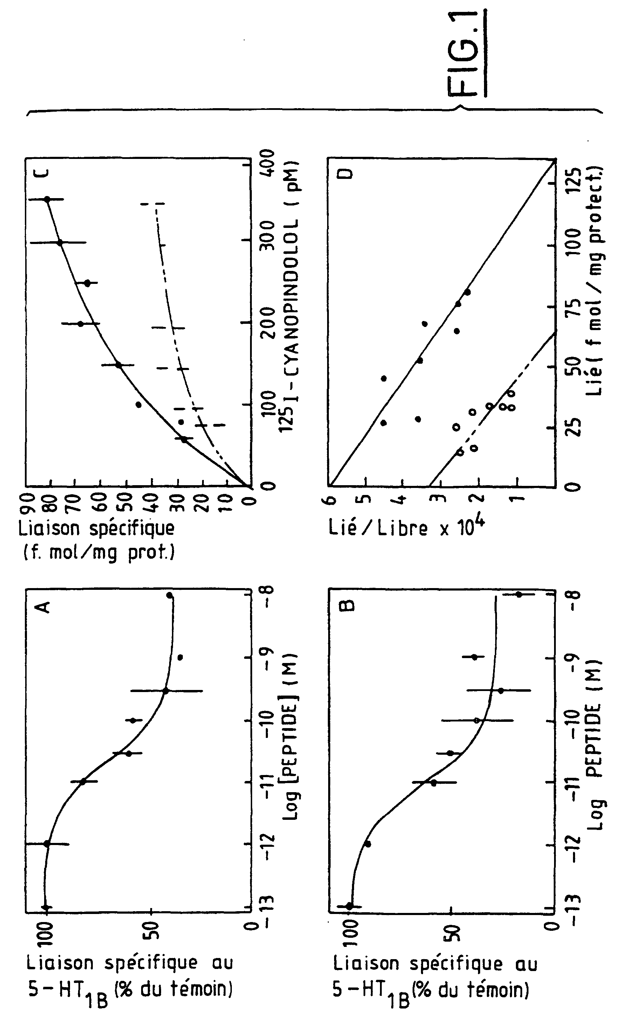

Cette dernière observation indique que les récepteurs 5-HT_{1B/1D} sont particulièrement sensibles au peptide. C'est pourquoi des essais ont été réalisés sur des homogénéisats de tissu cérébral de rat dans lesquels les récepteurs 5-HT_{1B} étaient marqués spécifiquement avec du [¹²⁵I] cyanopindolol, l'interaction correspondait à une CI₅₀ de 10⁻¹¹M et un effet maximum de 60%d'inhibition, comme représenté à la Figure 1A.

En outre, l'activité de ce peptide sur les récepteurs 5-HT_{1D} a été démontrée chez le cobaye et sur le cerveau humain à l'aide de [³H]5-HT. La valeur de la CI₅₀ était à nouveau de 10⁻¹¹M. Ces résultats suggèrent fortement que in vitro, les récepteurs 5-HT_{1B/1D} constituent la cible du peptide LSAL.

L'activité du peptide a également été évaluée sur la liaison aux récepteurs 5-HT_{1B} dans des cellules NIH 3T3 transfectées avec le gène du récepteur 5-HT_{1B} de la souris.

L'effet inhibiteur du peptide était très similaire à celui observé dans les homogénéisats de tissu cérébral (Figure 1B).

L'effet maximum correspondait à 70% d'inhibition de la liaison spécifique du [¹²⁵I] cyanopindolol avec une valeur de la CI₅₀ de 10⁻¹¹M. Ce résultat démontre clairement que LSAL interagit directement avec les récepteurs 5-HT_{1B}.

Pour étudier plus loin le type d'interaction du tétrapeptide avec les récepteurs 5-HT_{1B/1D}, des courbes de saturation du ligand radiomarqué ont été établies en présence du peptide. Les résultats de liaison obtenus suggèrent que l'activité inhibitrice du peptide correspond à une interaction non compétitive (Figures 1C, 1D).

Ce résultat a été observé avec la [³H]5-HT, aussi bien qu'avec le [¹²⁵I]cyanopindolol pour marquer les récepteurs 5-HT_{1B} et est en accord avec l'hypothèse précédente de l'existence d'un site spécifique pour le peptide distinct mais étroitement apparenté au site de liaison de 5-HT.

La spécificité pharmacologique des fractions partiellement purifiées a été étudiée au cours d'une purification ultérieure en utilisant des homogénéisats de cerveau de rat. Les essais ont été effectués en utilisant le LSAL synthétique pour déterminer si le peptide interagissait ou non avec d'autres récepteurs 5-HT, ou ceux d'autres neuro-transmetteurs.

Les récepteurs 5-HT _{1A} n'étaient pas affectés à une concentration du peptide qui antagonisait fortement la liaison au 5-HT_{1B/1D}.

De manière analogue, la liaison de radio-ligands aux récepteurs 5-HT_{1E}, 5-HT_{1F}, 5-HT₂ et 5-HT₃ n'était pas affectée (Tableau 2), ce qui indique que parmi les récepteurs sérotoninergiques étudiés, seule la classe 5-HT_{1B/1D} était sensible au peptide. La liaison d'autres radio-ligands spécifiques à des récepteurs de neurotransmetteurs variés a été examinée : les récepteurs α et β adrénergiques, dopaminergiques, muscariniques, histaminergiques, aux opiacées et aux benzodiazépines. La liaison à ces récepteurs n'était pas significativement réduite par LSAL à des concentrations qui avaient un effet inhibiteur maximum pour la liaison aux récepteurs 5-HT_{1B/1D} (Tableau 2).

**Tableau 2**

| **Effet inhibiteur du peptide sur différentes liaisons de radioligands dans des tissus de cerveau de rat** | | |
|---|---|---|
| **RECEPTEURS** | **LIGANDS** | **EFFET INHIBITEUR EN % DU TEMOIN** |
| **SEROTONINERGIQUES** | | |
| 5-HT_{1A} | [³H]8-OH-DPAT (3 nM) | 0 |
| 5-HT_{1B} | [¹²⁵I]cyanopindolol (0.3 nM) + 30 µM isoproterénol + 0,1 nM 8-OH DPAT | 70 ± 2 |
| 5-HT_{1 NONA} | [³H]5-HT (30 nM) + 0,1 µM 8-OH-DPAT | 75 ± 3 |
| 5-HT_{1EFC} | [³H]5-HT (30 nM) + 20 nM 5-CT | 0 ± 10 |
| 5-HT_{2A} | [³H]kétansérine (5 nM) | 10 ± 5 |
| 5-HT_{2A} | [³H]DOB (5 nM) | 2 ± 8 |
| 5-HT₃ | [³H]BRL 43694 (3 nM) | 0 ± 10 |

| **ADRENERGIQUES** | | |
|---|---|---|
| α₁ | [³H]prazosine (2 nM) | 5 ± 3 |
| β | [³H]dihydroalprénolol (3 nM) + 5-HT 10⁻⁵ M | 10 ± 15 |

| **DOPAMINERGIQUES** | | |
|---|---|---|
| **D**_{**2**} | [³H]spipérone (2 nM) + 10 µM 5-HT | 2 ± 3 |

| **HISTAMINERGIQUES** | | |
|---|---|---|
| **H**_{**1**} | [³H]mépyramine (5 nM) | 10 ± 10 |
| **MUSCARINIQUES** | [³H]benzylate de quinuclidinyle (3 nM) | 0 ± 2 |
| **OPIACEES** | [³H]naloxone (2 nM) | 10 ± 10 |
| **BENZODIAZEPINES** | [³H]flunitrazépam (3 nM) | 2 ± 2 |
| Légendes du Tableau 2 : | | |
| Des membranes cérébrales de cerveau de rat (200 µg) ont été incubées pendant 30 minutes à 25°C avec différents radio-ligands spécifiques (voir Tableau) en présence ou en absence de 1 nM du peptide. | | |
| Les différentes conditions de liaisons utilisées pour les récepteurs sérotoninergiques sont celles décrites précédemment (PALACIOS J.M. et al., Methods in Neurosciences 12, 238-262 (1993)). Pour les autres liaisons, le milieu d'incubation consistait en 50 mM Tris-HCl pH 7,4 contenant 120 mM de NaCl et 50 mM de KCl ([³H]spiropéridol, [³H] benzylate de quinuclidinyle, [³H]naloxone, ou 4 mM de CaCl₂ et 4 mM de MgCl₂ ([³H]flunitrazépam), ou 120 mM de NaCl, 5 mM de KCl, 2,5 mM de CaCl₂ et 1 mM de MgSO₄ ([³H]prazosin), ou 90 mM de NaCl ([³H]dihydroalprénolol). | | |
| Chaque point correspond à une moyenne plus ou moins l'écart type de cinq déterminations indépendantes. | | |

Le tétrapeptide identifié n'a pas montré d'autre effet sur la recapture de 5-HT dans les préparations synaptosomales. Il n'a pas non plus montré d'effet sur la recapture d'autres neurotransmetteurs (ou leurs précurseurs), par exemple la choline, le GABA, l'histamine, la dopamine et la noradrénaline. ce résultat indique que le peptide est différent des composés endogènes suspectés antérieurement d'interagir avec le transport des amines.

La liaison de la [³H]5-HT ou de [¹²⁵I]cyanopindolol sur les récepteurs de type 5-HT_{1B/1D} est également inhibée par les peptides suivants avec les CI₅₀ correspondantes :

- Ala - Leu   CI₅₀ = 10⁻⁶ M

- Leu- Ser  CI₅₀ = 10⁻⁵ M

- Ala - Leu - Ser   CI$_{50}$ = 10$^{-6}$ M

A titre comparatif, le peptide Ala - Leu-Leu - Ser s'est montré inactif dans le seul essai réalisé.

Le peptide Ala - Leu est actif dans sa forme L (forme naturelle) et ne présente quasiment pas d'activité dans sa forme D.

Les fractions Ala - Leu et Leu - Ser ont également montré une grande spécificité d'interaction, puisque leur effet inhibiteur de liaison ne s'exerçait que sur les sous-types 5-HT₁ des récepteurs 5-HT et plus particulièrement sur le sous-type 5-HT_{1B/1D} ; dans les conditions expérimentales utilisées, elles n'apparaissaient pas affecter les récepteurs 5-HT₂ et 5-HT₃ ni les récepteurs α adrénergiques, β adrénergiques, dopaminergiques, muscariniques, histaminergiques, récepteurs aux benzodiazépines et récepteurs aux opiacés. Ces peptides sont également dépourvus d'activité de déplacement du marqueur des sites de transport (³H paroxetine) et ne sont pas inhibiteurs de recapture de la 5-HT.

L'activité du peptide Ala - Leu a en outre été mesurée dans des essais préliminaires sur la fonction inhibitrice des récepteurs 5-HT_{1B/1D} sur la libération évoquée d'acétylcholine.

En accord avec les résultats des études de liaison indiquant une interaction avec les récepteurs 5-HT_{1B/1D}, il a été montré que le peptide LSAL antagonisait l'activité modulatrice des récepteurs 5-HT_{1B/1D} à très faible concentration et potentialisait au contraire l'effet d'un agoniste 5-HT_{1B} à concentration plus élevée. L'ensemble de ces résultats suggère que ce peptide pourrait jouer le rôle d'un modulateur allostérique du récepteur 5-HT_{1B/1D}.

### 3) Interaction de LSAL avec l'activité fonctionnelle des récepteurs 5-HT_{1B} (système de transduction)

Les récepteurs 5-HT_{1 B/D} sont connus pour être majoritairement couplés à la protéine Gi, en inhibant l'activité de l'adénylyl cyclase. L'interaction de LSAL a été examinée sur l'activité enzymatique corrélée à la stimulation du récepteur 5-HT_{1B} exprimée dans des cellules NIH 3T3.

Le peptide n'avait pas d'effet sur la production basale d'AMPc. Toutefois, il augmentait significativement l'effet inhibiteur de la 5-HT sur l'activité adénylyl cyclase stimulée par la forskoline (Figure 2). Cet effet était dépendant de la concentration avec une courbe dose-réponse en forme de cloche. L'activité du peptide sur les homogénéisats de substance noire de rat était pratiquement similaire. Ces résultats indiquent clairement que LSAL interagit avec l'activité du récepteur 5-HT_{1B}.

### 4) Effets du peptide sur l'activité fonctionnelle cellulaire des récepteurs 5-HT_{1B/D}

L'activité du peptide sur la libération de [³H]5-HT à partir de synaptosomes d'hippocampe, de cerveau de rat préalablement chargées en amine radioactive a été examinée.

La libération de [³H]5-HT induite par K⁺ a diminué en présence de LSAL de manière dose-dépendante avec une CI₅₀ apparente proche de 10⁻¹¹M. L'effet maximum, observé à une concentration de 10⁻¹⁰M, correspond à une inhibition de 20 à 25% de la libération évoquée et diminue à des concentrations plus élevées (Figure 3).

Dans des séries d'essais dont les conditions sont proches de celles utilisées pour les essais de liaison (à l'équilibre), le peptide LSAL ne potentialise plus l'action de la 5-HT mais au contraire s'oppose à son effet inhibiteur sur la libération de ³H-5HT.

Ces résultats démontrent que LSAL interagit avec la fonction cellulaire corrélée à l'activité des récepteurs 5-HT_{1B/1D}. L'hypothèse la plus vraisemblable pour expliquer ces résultats est que le peptide LSAL agissant en tant que modulateur allostérique du récepteur 5-HT_{1B/1D} est capable d'induire des transitions conformationnelles de ce récepteur correspondant soit à un état actif (potentialisation) soit à un état désensibilisé (inhibition) du récepteur.

### 5) Effets in vivo de LSAL

Des essais ont été réalisés in vivo chez la souris, pour examiner les effets comportementaux de LSAL.

Dans la mesure où des études neuro-pharmacologiques antérieures avaient montré l'implication des récepteurs 5-HT _{1B} dans les désordres liés au stress et à l'anxiété, les effets du peptide ont été examinés dans deux modèles animaux d'anxiété.

### Test du champ ouvert

L'activité de LSAL a été déterminée dans le test du champ ouvert sur des souris C57/Black-6.Des souris màles C57/BL/6 (20-25 g) ont été utilisées.Le champ ouvert consistait en une boîte de plastique blanc (35 x 35 x 20 cm) divisée en 25 carrés égaux. Du LSAL dissous dans 5 ml de 0,9% de solution saline dans un volume de 5 µl a été injecté par voie icv aux souris, qui ont été placées à un angle de la boîte. Après deux minutes, le nombre de carrés traversés, les demi-tours effectués et le temps d'immobilisation ont été mesurés. Les résultats sont exprimés comme la moyenne plus ou moins l'écart type. Différence avec le témoin : *p < 0,05, **p < 0,005 (Test de Student). A des doses de 50 µg ICV par animal, l'activité exploratrice a été significativement diminuée par rapport à des animaux témoins qui recevaient l'excipient dans les mêmes conditions (tableau 3).

**TABLEAU 3**

| **Effet d'injections intracérébroventriculaires de LSAL chez la souris dans le test du champ ouvert** | | | | |
|---|---|---|---|---|
| **Substance injectée** | **Nombre d'animaux** | **Nombre de carrés traversés** | **Nombre de redressements** | **Temps d'immobilisation (sec.)** |
| **Contrôle (NaCl)** | 12 | 115 ± 9 | 23 ± 3 | 12 ± 5 |
| **LSAL 50 µg** | 12 | 88 ± 9 * | 15 ± 3 * | 47 ± 8 ** |

### Labyrinthe en croix surélevé

Ce test expérimental est utilisé pour déterminer le degré d'anxiété et consiste en un labyrinthe surélevé à deux bras, l'un protégé par des parois et l'autre non protégé.

L'animal est supposé anxieux lorsqu'il évite l'entrée et réduit le temps passé dans les bras ouverts.

Des souris mâles BALB/C pesant de 20 à 24 g ont été utilisées.

Le labyrinthe en croix surélevé était fait de plexiglas avec bras ouverts et deux bras fermés de la même taille (12,5 x 5 cm) avec des murs de 15 cm de hauteur, ces bras partant d'une plateforme centrale de 5 x 5 cm, placée 40 cm au-dessus du sol.

Du LSAL dissous dans 5 ml de solution saline à 0,9% dans un volume de 5 µl a été injecté icv aux souris. Trois heures plus tard, l'animal a été placé sur la plateforme centrale du labyrinthe. Chaque entrée dans un bras ainsi que le temps passé dans chaque bras a été enregistré. La durée du test était de 5 minutes. Le labyrinthe a été nettoyé après chaque essai.

Le LSAL a été testé dans des conditions de basse intensité lumineuse (BENJAMIN D. et al., Life Sci. 47, 195-203 (1990)).

Les résultats ont été exprimés comme la moyenne plus ou moins l'écart type du pourcentage des entrées dans les bras ouverts (% d'entrées dans les bras ouverts), pourcentage du temps passé dans les bras ouverts (% temps dans le bras ouvert) et nombre total d'entrées dans les bras. Différence avec le témoin : *p < 0,05, **p < 0,01 (Test de Student).

**TABLEAU 4A**

| **(1 heure après injection i.v.)** | | | | |
|---|---|---|---|---|
| Substance injectée | Nombre d'animaux | % entrées dans les bras ouverts | %temps passé dans les bras ouverts | Entrées totales dans les bras |
| Témoin (NaCl) | 16 | 77 ±9 | 74±8 | 5±1 |
| LSAL 0,5 µg | 16 | 63±11 | 64±8 | 8±2 |
| LSAL 2 µg | 16 | 69±10 | 73±8 | 8±2 |
| LSAL 10 µg | 16 | 80±6 | 69±6 | 9±2* |

**TABLEAU 4B**

| **(3 heures après injection i.v.)** | | | | |
|---|---|---|---|---|
| Substance injectée | Nombre d'animaux | % entrées dans les bras ouverts | %temps passé dans les bras ouverts | Entrées totales dans les bras |
| Témoin (NaCl) | 35 | 62±5 | 71±5 | 8±1 |
| LSAL 2 µg | 6 | 60±8 | 62±9 | 15±2** |
| LSAL 10 µg | 16 | 80±5** | 85±4* | 14±1** |
| LSAL 50 µg | 26 | 56±7 | 55±7* | 8±1 |

Ce résultat suggère fortement que dans les conditions expérimentales mises en oeuvre, le peptide a un effet anxiogène. Une dose de 10 µg de LSAL augmente le nombre d'entrées dans les bras ouverts non protégés du labyrinthe, suggérant un effet anxiolytique de ce peptide dans les conditions d'essai.

### Test de la nage forcée

Le test de la nage forcée (Porsolt) est un test simple qui ne peut en aucun cas être assimilé à un modèle de "dépression". Il a cependant le mérite de discriminer un bon nombre de substances antidépressives. Il faut cependant remarquer que quelques antidépresseurs ne sont pas actifs dans ce test et certaines substances non antidépressives y sont actives.

Dans des conditions expérimentales où l'on mesure l'activité d'antidépresseurs dans ce test, il a été montré que le peptide Ala - Leu injecté par voie ip à forte concentration, mais aussi i.c.v. (intra cérébroventriculaire) à faible concentration, réduit légèrement mais significativement le temps d'immobilité des souris ; les antidépresseurs de type noradrénergique (nomifensine) sont beaucoup plus efficaces que le peptide dans ce test, mais les antidépresseurs sérotoninergiques ont également une activité faible.

La mesure de l'activité locomotrice horizontale a été réalisée dans un appareil d'activométrie. Le peptide a un effet stimulant. Au cours de ce test, une forte augmentation des défécations (signe lié à l'anxiété) a été notée chez des animaux qui ont reçu le peptide.

Cependant, dans tous ces essais, il semble que l'état de "stress" initial des animaux joue un rôle dans l'amplitude des phénomènes observés.

Diverses séries d'essais ont été réalisées sur les effets du dipeptide Ala - Leu sur l'appétit. Des administrations i.c.v. de 7 à 30 µg du peptide ont des effets très significatifs sur la prise de nourriture chez la souris, puisqu'elles entraînent sur 24 heures une diminution de près de la moitié de la consommation alimentaire des animaux, ainsi qu'une perte de poids également significative.

Ces dernières observations sont bien en accord avec le fait que les antidépresseurs sérotoninergiques ont le plus souvent des effets importants sur la prise alimentaire, certains favorisant, d'autres réduisant le poids chez les patients traités, et que leur activité est mise à profit dans les cas de boulimie ou d'anorexie qui souvent accompagnent la dépression.

Ces modifications comportementales observées après administration in vivo de LSAL et d'autres peptides selon l'invention montrent fortement que les modifications moléculaires et cellulaires induites par le peptide conduisent effectivement à des modifications de la fonction du système nerveux central. En fait, LSAL semble capable d'induire de l'anxiété après administration in vivo. LSAL apparaît comme un régulateur endogène, capable de moduler finement le contrôle exercé par la 5-HT sur la neurotransmission au niveau du système nerveux central. Il joue vraisemblablement un rôle-clé dans les fonctions physiologiques (sommeil, thermo-régulation, apprentissage et mémoire, comportement, douleur, ...), ainsi que dans des mécanismes physio-pathologiques (stress, anxiété, dépression, agressivité, désordres alimentaires, etc...) connus ou fortement soupçonnés pour impliquer le système sérotoninergique. Il devrait également jouer un rôle important dans les pathologies liées à la tension artérielle et la migraine, car des récepteurs de type 5-HT₁ (5-HT₁-like (probablement des 5-HT_{1D})) ont été localisés dans les vaisseaux de cerveau humain et dans d'autres muscles lisses.

Il régule sans doute également la réponse immune dans la mesure où les récepteurs 5-HT_{1B/1D} sont présents dans des tissus immuno-compétents, et dans la mesure où la transmission de la 5-HT est connue pour interagir avec la réponse immunitaire.

### 7) Activité relative des peptides selon l'invention

L'activité relative de différents aminoacides ou peptides à 1 nM a été déterminée sur des récepteurs 5-HT_{1B} et 5-HT_{1B/1D} à l'aide de 0,3 nM de [¹²⁵I] cyanopindolol et 30 nN [³H] 5-HT respectivement. Les expériences de liaison ont été réalisées comme décrit précédemment. Les résultats sont exprimés en pourcentage de l'effet de LSAL. Chaque valeur correspond à la moyenne ± l'écart type de trois essais indépendants réalisés en cinq exemplaires.

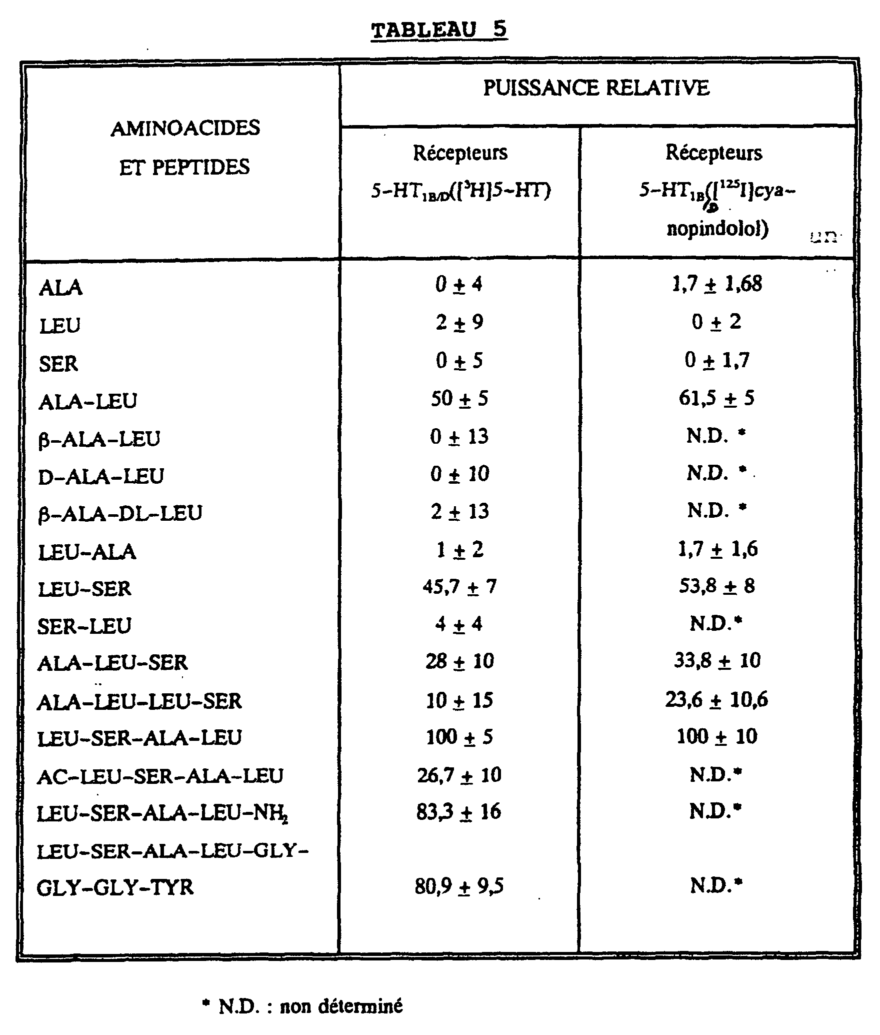

## Revendications

1. Composé de séquence peptidique : - X Leu Y dans laquelle X représente H ou Ala ou Leu-Ser-Ala, Y représente une séquence peptidique ayant de 1 à 10 aminoacides, dont l'extrémité carboxy terminale est amidifiée par un groupe NH₂ ou estérifiée par un reste hydroxycarbyloxy substitué ou non substitué, ou -Ala-Leu-Ser ou - Leu-Ser-Ala-Leu-Z dans laquelle Z représente OH, NH₂, un reste hydroxycarbyloxy substitué ou non substitué, ou une séquence peptidique ayant de 1 à 10 aminoacides,
- ainsi que les composés correspondants dans lesquels la liaison -CO-NH- peptidique est remplacée par une liaison résistante à la dégradation enzymatique des protéases ou dans lesquels le squelette peptidique comporte un ou plusieurs groupes intercalés rendant la liaison peptidique résistante à la dégradation enzymatique.

2. Composé de séquence peptidique : - X Leu Y dans laquelle X représente H ou Ala ou Leu-Ser-Ala, Y représente une séquence peptidique ayant de 1 à 10 aminoacides, dont l'extrémité carboxy terminale est amidifiée par un groupe NH₂ ou estérifiée par un reste hydroxycarbyloxy substitué ou non substitué,
à l'exclusion des composés peptidiques amidifiés de séquences Leu-Leu-NH₂, Leu-Leu-Leu-NH₂, et Leu-Leu-Phe-NH₂, - ou Ala-Leu-Ser - ou Leu-Ser-Ala-Leu-Z dans laquelle Z représente OH, NH₂, un reste hydroxycarbyloxy substitué ou non substitué, ou une séquence peptidique ayant de 1 à 10 aminoacides,
- ainsi que les composés correspondants dans lesquels la liaison -CO-NH- peptidique est remplacée par une liaison résistante à la dégradation enzymatique des protéases ou dans lesquels le squelette peptidique comporte un ou plusieurs groupes intercalés rendant la liaison peptidique résistante à la dégradation enzymatique.

3. Composés selon l'une des revendications 1 ou 2, dans lesquels le groupe de liaison peptidique -CO-NH- est remplacé par un groupe choisi parmi -CO-NR' ; -CR₁R₂-CR₃R₄-, -CO-CR₁R₂-, ou dans lesquels le squelette peptidique présente un ou plusieurs groupes intercalés choisis parmi les groupes -CR₁R₂-, -NR₁-, -O-, R₁, R₂, R₃ et R₄, identiques ou différents, représentant H ou un groupe alkyle en C₁-C₆, et R' représentant un groupe alkyle en C₁-C₆.

4. Composés selon l'une des revendications 1 ou 2, ayant une séquence choisie parmi : Leu-Ser-Ala-Leu Leu-Ser-Ala-Leu-OCH3 Leu-Ser-Ala-Leu-NH2 Leu-Ser-Ala-Leu-Gly-Gly-Gly-Tyr ainsi que les composés correspondants dans lesquels la liaison peptidique est remplacée par une liaison résistante à la dégradation enzymatique des protéases, ou dans lesquels le squelette peptidique comporte un ou plusieurs groupes intercalés rendant la liaison peptidique résistante à la dégradation enzymatique.

5. Composé selon l'une des revendications 1 à 4, **caractérisés en ce qu'**ils sont sous la forme L.

6. Procédé de production des composés tels que définis dans l'une quelconque des revendications 1 à 5, **caractérisé en ce que** l'on synthétise lesdits composés par synthèse peptidique classique, comme la synthèse en phase solide de Merrifield, lorsque lesdits composés sont de nature exclusivement peptidique, ou par modification des groupes terminaux des aminoacides et condensation des résidus ainsi obtenus, les fonctions réactives autres que celles engagées dans la liaison entre les résidus successifs ayant été préalablement protégées à l'aide de groupes protecteurs.

7. Composition thérapeutique, **caractérisée en ce qu'**elle comprend au moins un composé selon l'une quelconque des revendications 1 à 5.

8. Composition thérapeutique, **caractérisée en ce qu'**elle comprend une molécule précurseur susceptible de libérer *in vivo* un composé selon l'une quelconque des revendications 1 à 5.

9. Utilisation de composés de séquence peptidique : - X Leu Y dans laquelle X représente H ou Ala ou Leu-Ser-Ala, Y représente OH, ou une séquence peptidique ayant de 1 à 10 aminoacides, dont l'extrémité carboxy terminale est amidifiée par un groupe NH₂ ou estérifiée par un reste hydroxycarbyloxy substitué ou non substitué, sous réserve que lorsque X représente H, Y ne représente pas OH, ou -Ala-Leu-Ser, ou - Leu-Ser-Ala-Leu-Z dans laquelle Z représente OH, NH₂, un reste hydroxycarbyloxy substitué ou non substitué, ou une séquence peptidique ayant de 1 à 10 aminoacides,
- ainsi que les composés correspondants dans lesquels la liaison -CO-NH- peptidique est remplacée par une liaison résistante à la dégradation enzymatique des protéases ou dans lesquels le squelette peptidique comporte un ou plusieurs groupes intercalés rendant la liaison peptidique résistante à la dégradation enzymatique,
pour la préparation d'une composition ayant une activité modulatrice de la transmission sérotoninergique.

10. Utilisation de composés tels que définis dans la revendication 9 pour la préparation d'un médicament destiné au traitement d'une pathologie liée à une déficience de la transmission sérotoninergique.

11. Anticorps polyclonal ou monoclonal spécifiquement dirigé contre un peptide de séquence LSAL, ainsi que les fragments Fab, Fab', F(ab')₂ ou Fc.

12. Procédé de dosage immunologique d'un peptide LSAL dans un fluide biologique, dans lequel on met en présence un anticorps tel que défini dans la revendication 11 avec ledit fluide biologique et on met en évidence le complexe antigène LSAL-anticorps éventuellement formé.

13. Composition diagnostique comprenant un composé selon la revendication 1 ou 2 radiomarqué.

14. Procédé de visualisation des sites peptidiques des récepteurs 5-HT_{1B/1D}, **caractérisé en ce que** l'on utilise une composition selon la revendication 13, et que l'on visualise les récepteurs 5-HT_{1B/1D} à l'aide d'un scanner de tomographie par émission de positons.

15. Utilisation de composés tels que définis dans la revendication 9, pour cribler des molécules se fixant sur le récepteur 5-HT_{1B/1D} ou susceptibles de moduler la transmission sérotoninergique.

16. Procédé de criblage de composés susceptibles de moduler la transmission sérotoninergique, **caractérisé en ce qu'**on détermine:
i) la capacité de liaison des composés à tester sur le site de recapture de la 5-HT ; et
ii) la capacité de liaison des composés à tester sur le même site conformationnel du récepteur 5-HT_{1B/1D} que le peptide Leu-Ser-Ala-Leu ou qu'un composé tel que défini dans la revendication 9 ; les composés agissant sur le site 5-HT_{1B/1D} sans effet de recapture étant sélectionnés comme susceptibles de moduler la transmission sérotoninergique.

17. Procédé de mise en évidence de ligands du site du récepteur 5-HT_{1B/1D} pour le peptide LSAL, **caractérisé en ce que** l'on effectue les étapes consistant à :
- mettre en contact une molécule ou un mélange contenant différentes molécules, éventuellement non identifiées avec une cellule recombinée exprimant à sa surface le récepteur 5-HT_{1B/1D} en présence d'un composé tel que défini dans la revendication 9, dans des conditions permettant l'interaction entre le récepteur 5-HT_{1B/1D} et ladite(lesdites) molécule(s), dans le cas où celle-ci posséderait une affinité pour le récepteur 5-HT_{1B/1D} ;
- détecter la quantité dudit composé selon l'invention liée au récepteur ;
- en déduire la fixation éventuelle de ladite(desdites) molécule(s).

18. Procédé selon la revendication 16 ou 17 pour la mise en évidence d'agonistes ou d'antagonistes du peptide endogène LSAL.

19. Procédé de mise en évidence de modulateurs du site du récepteur 5-HT_{1B/1D} pour le peptide LSAL **caractérisé en ce que** l'on effectue les étapes consistant à :
- mettre en contact une molécule ou un mélange contenant différentes molécules, éventuellement non identifiées avec une cellule recombinée exprimant à sa surface le récepteur 5-HT_{1B/1D} en présence d'un composé tel que défini dans la revendication 9, dans des conditions permettant l'interaction entre le récepteur 5-HT_{1B/1D} et ledit composé tel que défini dans la revendication 8 ;
- détecter les molécules capables de moduler l'activité dudit composé tel que défini dans la revendication 9.

20. Ligand ou modulateur du site de récepteur 5-HT_{1B/1D} pour le peptide LSAL, identifié selon le procédé de l'une des revendications 16 à 19.

21. Composition thérapeutique comprenant comme principe actif un ligand ou modulateur selon la revendication 20.

22. Utilisation d'un ligand ou modulateur tel que défini dans la revendication 20 pour la préparation d'une composition ayant une activité modulatrice de la liaison sérotoninergique.

## Patentansprüche

1. Verbindung mit der Peptidsequenz -X Leu Y, in welcher X H, Ala oder Leu-Ser-Ala und Y eine Peptidsequenz mit 1 bis 10 Aminosäuren, deren terminale Carboxy-Gruppe durch eine NH₂-Gruppe amidiert bzw. durch einen gegebenenfalls substituierten Hydroxycarbyloxy-Rest verestert ist, bedeutet, -Ala-Leu-Ser oder -Leu-Ser-Ala-Leu-Z, wobei Z OH, NH₂, einen gegebenenfalls substituierten Hydroxycarbyloxy-Rest oder eine Peptidsequenz mit 1 bis 10 Aminosäuren bedeutet,
und die entsprechenden Verbindungen, in denen die -CO-NH-Peptidbindung durch eine Bindung ersetzt ist, die gegen einen enzymatischen Abbau durch Proteasen beständig ist, oder in denen das Peptidgerüst eine oder mehrere eingebaute Gruppen, welche die Peptidbindung gegen einen enzymatischen Abbau beständig machen, enthält.

2. Verbindung mit der Peptidsequenz -X Leu Y, in welcher X H, Ala oder Leu-Ser-Ala und Y eine Peptidsequenz mit 1 bis 10 Aminosäuren, deren terminale Carboxy-Gruppe durch eine NH₂-Gruppe amidiert bzw. durch einen gegebenenfalls substituierten Hydroxycarbyloxy-Rest verestert ist, bedeutet, wobei amidierte Peptidverbindungen mit den Sequenzen Leu-Leu-NH₂, Leu-Leu-Leu-NH₂ und Leu-Leu-Phe-NH₂ ausgeschlossen sind, -Ala-Leu-Ser oder -Leu-Ser-Ala-Leu-Z, wobei Z OH, NH₂, einen gegebenenfalls substituierten Hydroxycarbyloxy-Rest oder eine Peptidsequenz mit 1 bis 10 Aminosäuren bedeutet,
und die entsprechenden Verbindungen, in denen die -CO-NH-Peptidbindung durch eine Bindung ersetzt ist, die gegen einen enzymatischen Abbau durch Proteasen beständig ist, oder in denen das Peptidgerüst eine oder mehrere eingebaute Gruppen, welche die Peptidbindung gegen einen enzymatischen Abbau beständig machen, enthält.

3. Verbindungen nach Anspruch 1 oder 2, in welchen die Peptidbindungsgruppe -CO-NH- durch eine Gruppe ersetzt ist, die aus -CO-NR', -CR₁R₂-CR₃R₄- und -CO-CR₁R₂- ausgewählt ist, oder in welchen das Peptidgerüst eine oder mehrere eingebaute Gruppen aufweist, die aus den Gruppen -CR₁R₂-, -NR₁- und -O-ausgewählt sind, wobei R₁, R₂, R₃ und R₄ gegebenenfalls voneinander verschieden sind und H oder einen C₁- bis C₆-Alkylrest bedeuten und R' einen C₁- bis C₆-Alkylrest bedeutet.

4. Verbindungen nach Anspruch 1 oder 2, welche eine Sequenz besitzen, die ausgewählt ist aus Leu-Ser-Ala-Leu, Leu-Ser-Ala-Leu-OCH₃, Leu-Ser-Ala-Leu-NH₂, Leu-Ser-Ala-Leu-Gly-Gly-Gly-Tyr, und die entsprechenden Verbindungen, in denen die Peptidbindung durch eine Bindung ersetzt ist, die gegen einen enzymatischen Abbau durch Proteasen beständig ist, oder in denen das Peptidgerüst eine oder mehrere eingebaute Gruppen, welche die Peptidbindung gegen einen enzymatischen Abbau beständig machen, enthält.

5. Verbindungen nach einem der Ansprüche 1 bis 4,
**dadurch gekennzeichnet, dass** sie in der L-Form vorliegen.

6. Verfahren zur Herstellung der Verbindungen, wie sie in einem der Ansprüche 1 bis 5 definiert sind,
**dadurch gekennzeichnet, dass** die Verbindungen durch eine herkömmliche Peptidsynthese wie die Festphasentechnik Merrifield-Synthese, wenn der Charakter dieser Verbindungen ausschließlich peptidisch ist, oder durch Modifizierung der terminalen Gruppen der Aminosäuren und Kondensation der so erhaltenen Reste hergestellt werden, wobei die reaktiven Funktionen, die nicht an der Bindung zwischen den aufeinander folgenden Resten beteiligt sind, zuvor mit Schutzgruppen geschützt worden sind.

7. Therapeutische Zusammensetzung, **dadurch gekennzeichnet, dass** sie mindestens eine Verbindung gemäß einem der Ansprüche 1 bis 5 umfasst.

8. Therapeutische Zusammensetzung, **dadurch gekennzeichnet, dass** sie ein Vorläufermolekül umfasst, das in der Lage ist, *in vivo* eine Verbindung nach einem der Ansprüche 1 bis 5 abzuspalten.

9. Verwendung von Verbindungen mit der Peptidsequenz -X Leu Y, in welcher X H, Ala oder Leu-Ser-Ala und Y OH oder eine Peptidsequenz mit 1 bis 10 Aminosäuren, deren terminale Carboxy-Gruppe durch eine NH₂-Gruppe amidiert bzw. durch einen gegebenenfalls substituierten Hydroxycarbyloxy-Rest verestert ist, bedeutet, -Ala-Leu-Ser oder -Leu-Ser-Ala-Leu-Z, wobei Z OH, NH₂, einen gegebenenfalls substituierten Hydroxycarbyloxy-Rest oder eine Peptidsequenz mit 1 bis 10 Aminosäuren bedeutet,
und der entsprechenden Verbindungen, in welchen die Peptidbindung -CO-NH- durch eine Bindung ersetzt ist, die gegen einen enzymatischen Abbau durch Proteasen beständig ist, oder in denen das Peptidgerüst eine oder mehrere eingebaute Gruppen, welche die Peptidbindung gegen einen enzymatischen Abbau beständig machen, enthält,
zur Herstellung einer Zusammensetzung mit einer Aktivität, welche die serotoninerge Übertragung moduliert.

10. Verwendung von Verbindungen wie in Anspruch 9 definiert zur Herstellung eines Arzneimittels für die Behandlung einer Erkrankung, die mit einem Defizit in der serotoninergen Übertragung verbunden ist.

11. Polyklonaler oder monoklonaler Antikörper, der spezifisch gegen ein Peptid mit der Sequenz LSAL gerichtet ist, und die Fragmente Fab, Fab', F(ab')₂ oder Fc.

12. Verfahren zur quantitativen immunologischen Bestimmung eines LSAL-Peptids in einem biologischen Fluid, bei welchem ein wie in Anspruch 11 definierter Antikörper mit dem biologischen Fluid in Berührung gebracht und der gegebenenfalls gebildete LSAL-Antigen-Antikörper-Komplex nachgewiesen wird.

13. Diagnostische Zusammensetzung, die eine radioaktiv markierte Verbindung nach Anspruch 1 oder 2 umfasst.

14. Verfahren zur Sichtbarmachung der Peptiderkennungsstellen der 5-HT_{1B/1D}-Rezeptoren,
**dadurch gekennzeichnet, dass** eine Zusammensetzung nach Anspruch 13 verwendet wird und die 5-HT_{1B/1D}-Rezeptoren durch Positronenemissionstomographie sichtbar gemacht werden.

15. Verwendung von Verbindungen wie in Anspruch 9 definiert zum Scannen von Molekülen, die sich an den 5-HT_{1B/1D}-Rezeptor anheften oder in der Lage sind, die serotoninerge Übertragung zu modulieren.

16. Verfahren zum Scannen von Verbindungen, die in der Lage sind, die serotoninerge Übertragung zu modulieren, **dadurch gekennzeichnet, dass**
I) das Vermögen der zu untersuchenden Verbindungen, an die Wiederaufnahmestelle für das 5-HT zu binden, und
II) das Vermögen der zu untersuchenden Verbindungen, an dieselbe Konformationsstelle des 5-HT_{1B/1D}-Rezeptors wie das Leu-Ser-Ala-Leu-Peptid oder eine wie in Anspruch 9 defnierte Verbindung zu binden,
bestimmt wird, wobei die Verbindungen, die auf die 5-HT_{1B/1D}-Erkennungsstelle ohne Wiederaufnahmeeffekt wirken, als fähig, die serotoninerge Übertragung zu modulieren, ausgewählt werden.

17. Verfahren zum Nachweis von Liganden an der Erkennungsstelle des 5-HT_{1B/1D}-Rezeptors für das LSAL-Peptid, **dadurch gekennzeichnet, dass** die Stufen durchgeführt werden, die aus
- In-Berührung-Bringen eines Moleküls oder eines Gemischs, das gegebenenfalls nicht identifizierte unterschiedliche Moleküle enthält, mit einer rekombinierten Zelle, die an ihrer Oberfläche den 5-HT_{1B/1D}-Rezeptor exprimiert, in Gegenwart einer wie in Anspruch 9 definierten Verbindung unter Bedingungen, welche die Wechselwirkung des 5-HT_{1B/1D}-Rezeptors mit dem/den Molekül/en dann erlauben, wenn diese/s eine Affinität für den 5-HT_{1B/1D}-Rezeptor besitzt/besitzen,
- Detektieren der Menge der an den Rezeptor gebundenen erfindungsgemäßen Verbindung und
- daraus Ableiten der eventuellen Fixierung dieses/dieser Moleküls/Moleküle
bestehen.

18. Verfahren nach Anspruch 16 oder 17 zum Nachweis von Agonisten oder Antagonisten des endogenen LSAL-Peptids.

19. Verfahren zum Nachweis von Modulatoren der Erkennungsstelle des 5-HT_{1B/1D}-Rezeptors für das LSAL-Peptid, **dadurch gekennzeichnet, dass** die Stufen durchgeführt werden, die aus
- In-Berührung-Bringen eines Moleküls oder eines Gemischs, das gegebenenfalls nicht identifizierte unterschiedliche Moleküle enthält, mit einer rekombinierten Zelle, die an ihrer Oberfläche den 5-HT_{1B/1D}-Rezeptor exprimiert, in Gegenwart einer wie in Anspruch 9 definierten Verbindung unter Bedingungen, welche die Wechselwirkung des 5-HT_{1B/1D}-Rezeptors mit der in Anspruch 8 definierten Verbindung erlauben, und
- Detektieren der Moleküle, die in der Lage sind, die Aktivität der in Anspruch 9 definierten Verbindung zu modulieren,
bestehen.

20. Ligand oder Modulator der Erkennungsstelle des 5-HT_{1B/1D}-Rezeptors für das LSAL-Peptid, der gemäß dem Verfahren nach einem der Ansprüche 16 bis 19 identifziert worden ist.

21. Therapeutische Zusammensetzung, die als Wirkstoff einen Liganden oder Modulator gemäß Anspruch 20 enthält.

22. Verwendung eines wie in Anspruch 20 definierten Liganden oder Modulators zur Herstellung einer Zusammensetzung, die eine die serotoninerge Bindung modulierende Aktivität besitzt.

## Claims

1. Compound having the peptide sequence: - X Leu Y
in which X represents H or Ala or Leu-Ser-Ala, Y represents a peptide sequence having from 1 to 10 amino acids, the terminal carboxy end of which has been amidated by an NH₂ group or esterified by a substituted or unsubstituted hydroxycarbyloxy radical,
or -Ala-Leu-Ser or -Leu-Ser-Ala-Leu-Z
in which Z represents OH, NH₂, a substituted or unsubstituted hydroxycarbyloxy radical, or a peptide sequence having from 1 to 10 amino acids,
- and the corresponding compounds in which the -CO-NH- peptide bond has been replaced by a bond that is resistant to protease enzymatic degradation or in which the peptide skeleton has one or more intercalated groups rendering the peptide bond resistant to enzymatic degradation.

2. Compound having the peptide sequence: - X Leu Y in which X represents H or Ala or Leu-Ser-Ala, Y represents a peptide sequence having from 1 to 10 amino acids, the terminal carboxy end of which has been amidated by an NH₂ group or esterified by a substituted or unsubstituted hydroxycarbyloxy radical, with the exception of the amidated peptide compounds having the sequences Leu-Leu-NH₂, Leu-Leu-Leu-NH₂ and Leu-Leu-Phe-NH₂,
- or Ala-Leu-Ser - or Leu-Ser-Ala-Leu-Z in which Z represents OH, NH₂, a substituted or unsubstituted hydroxycarbyloxy radical, or a peptide sequence having from 1 to 10 amino acids,
- and the corresponding compounds in which the -CO-NH- peptide bond has been replaced by a bond that is resistant to protease enzymatic degradation or in which the peptide skeleton has one or more intercalated groups rendering the peptide bond resistant to enzymatic degradation.

3. Compounds according to either claim I or claim 2, in which the peptide bonding group -CO-NH- has been replaced by a group selected from -CO-NR'; -CR₁R₂-CR₃R₄-, -CO-CR₁R₂-, or in which the peptide skeleton has one or more intercalated groups selected from the groups -CR₁R₂-, -NR₁-, -O-, wherein R₁, R₂, R₃ and R₄, which may be identical or different, represent H or a C₁-C₆alkyl group, and R' represents a C₁-C₆alkyl group.

4. Compounds according to either claim 1 or claim 2, having a sequence selected from: Leu-Ser-Ala-Leu Leu-Ser-Ala-Leu-OCH3 Leu-Ser-Ala-Leu-NH2 Leu-Ser-Ala-Leu-Gly-Gly-Gly-Tyr and the corresponding compounds in which the peptide bond has been replaced by a bond that is resistant to protease enzymatic degradation or in which the peptide skeleton has one or more intercalated groups rendering the peptide bond resistant to enzymatic degradation.

5. Compound according to any one of claims 1 to 4, **characterised in that** they are in the L form.

6. Process for the production of compounds as defined in any one of claims I to 5, **characterised in that** the said compounds are synthesised by conventional peptide synthesis, such as Merrifield solid-phase synthesis, when said compounds are of a solely peptide nature, or by modification of the terminal groups of the amino acids and condensation of the residues so obtained, the reactive functions other than those involved in the bond between successive residues having previously been protected by means of protecting groups.

7. Therapeutic composition, **characterised in that** it comprises at least one compound according to any one of claims 1 to 5.

8. Therapeutic composition, **characterised in that** it comprises a precursor molecule capable of liberating *in vivo* a compound according to any one of claims 1 to 5.

9. Use of compounds having the peptide sequence: - X Leu Y
in which X represents H or Ala or Leu-Ser-Ala, Y represents OH or a peptide sequence having from 1 to 10 amino acids, the terminal carboxy end of which has been amidated by an NH₂ group or esterified by a substituted or unsubstituted hydroxycarbyloxy radical,
with the proviso that, when X represents H, Y does not represent OH,
or -Ala-Leu-Ser
or -Leu-Ser-Ala-Leu-Z
in which Z represents OH, NH₂, a substituted or unsubstituted hydroxycarbyloxy radical, or a peptide sequence having from 1 to 10 amino acids,
- and the corresponding compounds in which the -CO-NH- peptide bond has been replaced by a bond that is resistant to protease enzymatic degradation or in which the peptide skeleton has one or more intercalated groups rendering the peptide bond resistant to enzymatic degradation, in the preparation of a composition having an activity modulating serotoninergic transmission.

10. Use of compounds as defined in claim 9 in the preparation of a medicament for the treatment of a pathology associated with a deficiency in serotoninergic transmission.

11. Polyclonal or monoclonal antibody directed specifically against a peptide having the sequence LSAL, as well as the fragments Fab, Fab', F(ab')₂ or Fc.

12. Method for the immunological determination of a peptide LSAL in a biological fluid, in which an antibody as defined in claim 11 is brought together with said biological fluid and the LSAL-antibody antigen complex that may form is demonstrated.

13. Diagnostic composition comprising a radiolabelled compound according to either claim 1 or claim 2.

14. Method of indicating visually the peptide sites of 5-HT_{1B/1D} receptors, **characterised in that** a composition according to claim 13 is used, and **in that** the 5-HT_{1B/1D} receptors are indicated visually with the aid of a positron-emission tomography scanner.

15. Use of compounds as defined in claim 9 for screening molecules that attach themselves to the 5-HT_{1B/1D} receptor or are capable of modulating serotoninergic transmission.

16. Method of screening compounds capable of modulating serotoninergic transmission, **characterised in that** there are determined:
i) the capacity of the compounds to be tested to bind to the site of 5-HT recapture; and
ii) the capacity of the compounds to be tested to bind to the same conformational site of the 5-HT_{1B/1D} receptor as the peptide Leu-Ser-Ala-Leu or as a compound as defined in claim 9;
the compounds that act on the 5-HT_{1B/1D} site without a recapture effect being selected as capable of modulating serotoninergic transmission.

17. Method of demonstrating ligands of the 5-HT_{1B/1D} receptor site for the peptide LSAL, **characterised in that** there are carried out steps consisting in:
- bringing a molecule or a mixture containing various, optionally unidentified, molecules together with a recombined cell expressing at its surface the 5-HT_{1B/1D} receptor in the presence of a compound as defined in claim 9, under conditions permitting interaction between the 5-HT_{1B/1D} receptor and said molecule(s) in the case where the latter has affinity for the 5-HT_{1B/1D} receptor;
- detecting the amount of said compound according to the invention bonded to the receptor;
- deriving therefrom the optional fixing of said molecule(s).

18. Method according to either claim 16 or claim 17 for demonstrating agonists or antagonists of the endogenous peptide LSAL.

19. Method of demonstrating modulators of the 5-HT_{1B/1D} receptor site for the peptide LSAL, **characterised in that** there are carried out steps consisting in:
- bringing a molecule or a mixture containing various, optionally unidentified, molecules together with a recombined cell expressing at its surface the 5-HT_{1B/1D} receptor in the presence of a compound as defined in claim 9, under conditions permitting interaction between the 5-HT_{1B/1D} receptor and said compound as defined in claim 8;
- detecting molecules capable of modulating the activity of said compound as defined in claim 9.

20. Ligand or modulator of the 5-HT_{1B/1D} receptor site for the peptide LSAL, identified by the method of any one of claims 16 to 19.

21. Therapeutic composition comprising as active ingredient a ligand or modulator according to claim 20.

22. Use of a ligand or modulator as defined in claim 20 in the preparation of a composition having an activity modulating the serotoninergic bond.
